# EUROPEAN PATENT APPLICATION

(11) **EP 4 202 057 A1**
(43) Date of publication of application: **28.06.2023**
(21) Application number: 21217571.5
(22) Date of filing: 23.12.2021
(51) Int. Cl.: C12Q 1/686, C12Q 1/6886

(54) **DETECTION OF TELOMERE FUSION EVENTS**

(71) Applicant: European Molecular Biology Laboratory, 69117 Heidelberg (DE); Centro Nacional de Investigaciones Cardiovasculares Carlos III F.S.P. (CNIC), 28029 Madrid (ES); Consejo Superior de Investigaciones Científicas (CSIC), 28006 Madrid (ES)
(72) Inventor: Cortes-Ciriano, Isidro, Cambridgeshire, CB10 1SD (GB); Muyas-Remolar, Francesc, Cambridgeshire, CB10 1SD (GB); Flores Hernández, Ignacio, 28049 Madrid (ES); Gómez Rodríguez, Manuel José, 28029 Madrid (ES)
(74) Representative: Kuttenkeuler, David

(57) **Abstract**

The invention pertains to means and methods for the detection of telomere fusion events, and the use of such means and methods in the detection and diagnosis of a disease associated with telomere fusion events, such as a cancer disease.

## Description

### FIELD OF THE INVENTION

The invention pertains to means and methods for the detection of telomere fusion events, and the use of such means and methods in the detection and diagnosis of a disease associated with telomere fusion events, such as a cancer disease.

### DESCRIPTION

Telomeres are nucleoprotein complexes composed of telomeric TTAGGG repeats and telomere binding proteins that prevent the recognition of chromosome ends as sites of DNA damage¹. The replicative potential of somatic cells is limited by the length of telomeres, which shorten at every cell division due to end-replication losses. Most human cancers acquire replicative immortality by re-expressing telomerase through diverse mechanisms², including activating TERT promoter mutations^{3,4} and enhancer hijacking⁵. In other cancers, in particular those of mesenchymal or neuroendocrine origin, telomeres are elongated by the alternative lengthening of telomeres (ALT) pathway, which relies on recombination⁶. Telomere attrition can result in senescence or the ligation of chromosome ends to form dicentric chromosomes, which are observed as chromatin bridges during anaphase⁷. The resolution of chromosome bridges caused by telomere fusions (TFs) can increase genomic complexity and the acquisition of oncogenic alterations involved in malignant transformation and resistance to chemotherapy through diverse mechanisms, including chromothripsis and breakage-fusion-bridge cycles⁸⁻¹².

Despite their importance in tumour evolution, the patterns and consequences of TFs remain largely uncharacterized, in part due to technical challenges. TFs have been traditionally detected by inspection of chromosome bridges in metaphase spreads¹³⁻¹⁵. In recent years, the study of TFs has relied on PCR-based methods using primers annealing to a subset of subtelomeric regions^{16,17} which are limited to detect TFs distantly located from subtelomeres since PCR efficiency decreases as the amplicon size increases¹⁸. To overcome these limitations, the inventors have developed analytical methods to detect TFs using whole-genome sequencing (WGS) data.

There is still an unmet need for a quick identification of the presence of cancerous markers in humans in order to allow early disease diagnosis and treatment.

### BRIEF DESCRIPTION OF THE INVENTION

Generally, and by way of brief description, the main aspects of the present invention can be described as follows:

In **a first aspect,** the invention pertains to a method for the detection of a telomere fusion event, the method comprising a step of detecting the presence or absence of a nucleic acid sequence comprising a first sequence stretch and a second sequence stretch on the same nucleic acid strand, wherein
- the first sequence-stretch is a sequence of at least 12 directly adjacent nucleic acid base pairs (bp) within the sequence: GGGTTAGGGTTAGGGTTA (SEQ ID NO: 1), wherein the first sequence stretch may not comprise more than two, preferably no more than one, bp variation within this sequence;
- the second sequence-stretch is a sequence of at least 12 directly adjacent nucleic acid bp within the sequence: CCCTAACCCTAACCCTAA (SEQ ID NO: 2), wherein the second sequence stretch may not comprise more than two, preferably no more than one, bp variation within this sequence;
wherein the presence of the at least one indicator nucleic acid sequence the presence of the at least one telomere fusion event.

In **a second aspect,** the invention pertains to a method for the detection of the presence of at least one telomere fusion event, the method comprising the steps of:
- Providing a dataset of nucleic acid sequencing reads, wherein the dataset of nucleic acid sequencing reads is obtained by next generation sequencing (NGS) or long-read sequencing of nucleic acids of nucleic acids derived from a cellular sample;
- Detecting within the dataset of nucleic acid sequencing reads the presence or absence of at least one indicator sequencing read which is characterized by having a nucleic acid sequence comprising a first sequence stretch and a second sequence stretch on the same strand, wherein:
   - the first sequence-stretch is a sequence of at least 12 directly adjacent nucleic acid base pairs (bp) within the sequence: GGGTTAGGGTTAGGGTTA (SEQ ID NO: 1), wherein the first sequence stretch may not comprise more than two, preferably no more than one, bp variation within this sequence;
   - the second sequence-stretch is a sequence of at least 12 directly adjacent nucleic acid bp within the sequence: CCCTAACCCTAACCCTAA (SEQ ID NO: 2), wherein the second sequence stretch may not comprise more than two, preferably no more than one, bp variation within this sequence;
wherein the presence of the at least one indicator nucleic acid sequencing read indicates the presence of the at least one telomere fusion event.

In **a third aspect,** the invention pertains to a computer readable medium comprising computer readable instructions stored thereon that when run on a computer perform a method according to the invention.

In **a fourth aspect,** the invention pertains a method for the diagnosis of a cancer disease in a subject, comprising the steps of detecting the absence or presence of a telomere fusion event in a sample of the subject using a method of the invention for the detection of a telomere fusion event according to the previous aspects.

In **a fifth aspect,** the invention pertains a method for the diagnosis of a cancer disease in a subject, comprising the steps of
- Providing a biological sample of the subject to be diagnosed;
- Nucleic acid sequencing the biological sample to obtain a dataset of nucleic acid sequencing reads;
- Performing a method according to the invention for the detection of a telomere fusion event with the dataset of nucleic acid sequencing reads of (b) in order to detect the presence or absence of at least one indicator sequencing read in the dataset of nucleic acid sequencing reads;

Wherein the presence of the at least one indicator sequencing read indicates the presence of a cancer disease characterized by the presence of a telomere fusion event in the subject.

### DETAILED DESCRIPTION OF THE INVENTION

In the following, the elements of the invention will be described. These elements are listed with specific embodiments, however, it should be understood that they may be combined in any manner and in any number to create additional embodiments. The variously described examples and preferred embodiments should not be construed to limit the present invention to only the explicitly described embodiments. This description should be understood to support and encompass embodiments which combine two or more of the explicitly described embodiments or which combine the one or more of the explicitly described embodiments with any number of the disclosed and/or preferred elements. Furthermore, any permutations and combinations of all described elements in this application should be considered disclosed by the description of the present application unless the context indicates otherwise.

In **a first aspect,** the invention pertains to a method for the detection of a telomere fusion event, the method comprising a step of detecting the presence or absence of a nucleic acid sequence comprising a first sequence stretch and a second sequence stretch on the same nucleic acid strand, wherein
- the first sequence-stretch is a sequence of at least 12 directly adjacent nucleic acid base pairs (bp) within the sequence: GGGTTAGGGTTAGGGTTA (SEQ ID NO: 1), wherein the first sequence stretch may not comprise more than two, preferably no more than one, bp variation within this sequence;
- the second sequence-stretch is a sequence of at least 12 directly adjacent nucleic acid bp within the sequence: CCCTAACCCTAACCCTAA (SEQ ID NO: 2), wherein the second sequence stretch may not comprise more than two, preferably no more than one, bp variation within this sequence;
wherein the presence of the at least one indicator nucleic acid sequence the presence of the at least one telomere fusion event.

In context of the present invention, it was discovered that a telomere fusion event can be detected by determining the presence or absence of one nucleic acid sequence stretch that is found in inward or outward fusion events. Throughout the present disclosure the nucleic acid to be detected is generally referred to as an "indicator nucleic acid" or, in case the invention pertains to a next generation sequencing approach, also referred to as "indicator sequencing read". Such an indicator shall be understood to contain on one strand a first and a second sequence stretch - which may be present in any sequence - and which are defined as follows:

The first sequence-stretch is a sequence of at least 12 directly adjacent nucleic acid base pairs (bp) within the sequence: GGGTTAGGGTTAGGGTTA (SEQ ID NO: 1), wherein the first sequence stretch may not comprise more than two, preferably no more than one, bp variation within this sequence.

The second sequence-stretch is a sequence of at least 12 directly adjacent nucleic acid bp within the sequence: CCCTAACCCTAACCCTAA (SEQ ID NO: 2), wherein the second sequence stretch may not comprise more than two, preferably no more than one, bp variation within this sequence.

The indicator sequences of the invention in some preferred alternative embodiments is a sequence of at least 12 closely adjacent nucleic acid bp within the sequence of either SEQ ID NO: 1 or 2 as described above, wherein closely adjacent shall comprise sequence stretches with not more than 102 separating nucleic acid positions, preferably wherein not more than one or two of any 5 bp long repeating unit within SEQ ID NO 1 or 2 contain a separating nucleic acid position. A separating nucleic acid position shall be understood as a position within the sequence that constitutes an irregularity within the repetition pattern of the sequences of SEQ ID NO: 1 or 2, respectively.

The indicator sequence or indicator nucleic acid may be detected in accordance with the invention with any means available to the skilled artisan that allows a sequence specific detection of the indicator nucleic acid. Such procedures are generally referred to as "nucleic acid detection assay", and the term shall be understood to refers to any method of determining the nucleotide composition of a nucleic acid of interest. Nucleic acid detection assays include but are not limited to, DNA sequencing methods, in particular next generation sequencing (NGS), probe hybridization methods, enzyme mismatch cleavage methods; polymerase chain reaction (PCR), and PCR based assays; branched hybridization methods; rolling circle replication; any other Nucleic acid sequence-based amplification; ligase chain reaction; and sandwich hybridization methods, and any combination thereof.

Thus, the present invention shall in addition pertain to any nucleic acid primer probe that specifically hybridizes to an indicator of the invention and, thus, is useful in the any of the aspects of the present invention.

The term "primer" refers to an oligonucleotide, whether occurring naturally as in a purified restriction digest or produced synthetically, that is capable of acting as a point of initiation of synthesis when placed under conditions in which synthesis of a primer extension product that is complementary to a nucleic acid strand is induced, (e.g., in the presence of nucleotides and an inducing agent such as a DNA polymerase and at a suitable temperature and pH). The primer is preferably single stranded for maximum efficiency in amplification but may alternatively be double stranded. If double stranded, the primer is first treated to separate its strands before being used to prepare extension products. Preferably, the primer is an oligodeoxyribonucleotide. The primer must be sufficiently long to prime the synthesis of extension products in the presence of the inducing agent. The exact lengths of the primers will depend on many factors, including temperature, source of primer, and the use of the method.

The term "probe" refers to an oligonucleotide (e.g., a sequence of nucleotides), whether occurring naturally as in a purified restriction digest or produced synthetically, recombinantly, or by PCR amplification, that is capable of hybridizing to another oligonucleotide of interest, such as an indicator nucleic acid of the invention. A probe may be single-stranded or double-stranded. Probes are useful in the detection, identification, and isolation of particular gene sequences (e.g., a "capture probe"). It is contemplated that any probe used in the present invention may, in some embodiments, be labeled with any "reporter molecule," so that is detectable in any detection system, including, but not limited to enzyme (e.g., ELISA, as well as enzyme-based histochemical assays), fluorescent, radioactive, and luminescent systems. It is not intended that the present invention be limited to any particular detection system or label. A probe in context of the invention is preferably designed such that is specifically detects the presence or absence of an indicator nucleic acid. Such a probe specifically hybridizes to an indicator nucleic acid, and not to a nucleic acid sequence that contains only the first or the second sequence stretch.

The term "sample" is used in its broadest sense. In one sense it can refer to an animal cell or tissue. In another sense, it is meant to include a specimen or culture obtained from any source, as well as other biological samples. Biological samples may be obtained from plants or animals (including humans) and encompass fluids (e.g., urine, blood, etc.), solids, tissues, and gases. These examples are not to be construed as limiting the sample types applicable to the present invention. Preferably a sample is a biological sample and contains nucleic acid material of chromosomes, or nucleic acid material that is derived from chromosomes, such as extra chromosomal nucleic acids.

As used herein, the term "extra-chromosomal nucleic acids" means any nucleic acid that may be found in a biological sample that is not part of the chromosomal material of a cell, i.e. not genomic DNA. Examples of extra-chromosomal nucleic acids contain any fragmented genomic material.

As used herein, the terms "patient" or "subject" refer to organisms to be subject to various tests provided by the technology. The term "subject" includes animals, preferably mammals, including humans. In a preferred embodiment, the subject is a primate. In an even more preferred embodiment, the subject is a human. In typical embodiments, a subject is a female.

In **a second aspect,** the invention pertains to a method for the detection of the presence of at least one telomere fusion event, the method comprising the steps of:
- Providing a dataset of nucleic acid sequencing reads, wherein the dataset of nucleic acid sequencing reads is obtained by next generation sequencing (NGS) or long-read sequencing of nucleic acids of nucleic acids derived from a cellular sample;
- Detecting within the dataset of nucleic acid sequencing reads the presence or absence of at least one indicator sequencing read which is characterized by having a nucleic acid sequence comprising a first sequence stretch and a second sequence stretch on the same strand, wherein:
   - the first sequence-stretch is a sequence of at least 12 directly adjacent nucleic acid base pairs (bp) within the sequence: GGGTTAGGGTTAGGGTTA (SEQ ID NO: 1), wherein the first sequence stretch may not comprise more than two, preferably no more than one, bp variation within this sequence;
   - the second sequence-stretch is a sequence of at least 12 directly adjacent nucleic acid bp within the sequence: CCCTAACCCTAACCCTAA (SEQ ID NO: 2), wherein the second sequence stretch may not comprise more than two, preferably no more than one, bp variation within this sequence;
wherein the presence of the at least one indicator nucleic acid sequencing read indicates the presence of the at least one telomere fusion event.

The second aspect therefore shall be understood as a specific embodiment of the first aspect using the NGS.

In preferred embodiments of the invention the indicator nucleic acid or indicator nucleic acid sequencing read is further characterized in that the first sequence-stretch and second sequence-stretch are directly adjacent to each other, or are separated by an inserted sequence having a length of 1 to 100 nucleic acids, or 1 to 50, preferably about 5, 10, 15, 20, 25, 30, 40, 50, 60, 70, 80, 90 or 100 nucleic acids.

In a preferred embodiment of the invention, if the indicator nucleic acid or indicator nucleic acid sequencing read is further characterized in that the first sequence stretch is in 5' position of the second sequence stretch, the presence of the at least one indicator nucleic acid or indicator nucleic acid sequencing read indicates the presence of the at least one inward telomere fusion event. Alternatively, if the indicator nucleic acid or indicator nucleic acid sequencing read is further characterized in that the first sequence stretch is in 3' position of the second sequence stretch, the presence of the at least one indicator nucleic acid or indicator nucleic acid sequencing read indicates the presence of the at least one outward telomere fusion event.

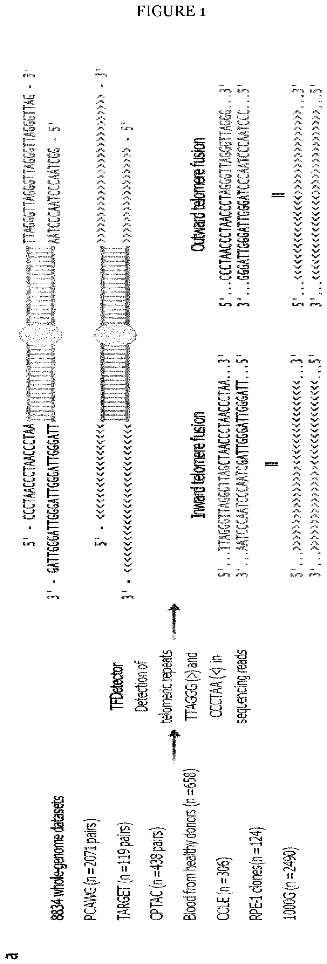

The term "inward telomere fusion" or an "outward telomere fusion" shall denote a telomere fusion event according to the illustration in figure 1a.

If, in accordance with the invention, the detection is performed using NGS; then the obtained dataset of nucleic acid sequencing reads may preferably have a coverage of at least 0,1×, preferably 1×, 5×, 10×, preferably at least 50× more preferably of about 100×. The dataset of nucleic acid sequencing reads may be obtained from a sample comprising multiple cells of the same type, or may comprise nucleic acids from a variety of sources.

The method of any one of claims 1 to 5, wherein the method is for the detection of the presence of a telomere fusion event in a cell, which can be a healthy or cancerous cell, and wherein the dataset of nucleic acid sequencing reads is derived from genomic material of the cell.

A telomere fusion in accordance with the invention is in some embodiments a telomere fusion of the alternative lengthening of telomeres (ALT-TF).

The method of the invention may be an in-vitro and/or in-silico method.

As used herein, the term "specificity" is the percentage of subjects correctly identified as having a particular disease i.e., normal or healthy subjects. For example, the specificity is calculated as the number of subjects with a particular disease as compared to non-cancer subjects (e.g., normal healthy subjects).

By "specifically binds" is meant a compound such as a nucleic acid probe that recognizes and binds an indicator of the invention.

Nucleic acid molecules useful in the methods of the invention include any nucleic acid molecule that encodes a polypeptide of the invention or a fragment thereof. Such nucleic acid molecules need not be 100% identical with an endogenous nucleic acid sequence, but will typically exhibit substantial identity. Polynucleotides having "substantial identity" to an endogenous sequence are typically capable of hybridizing with at least one strand of a double-stranded nucleic acid molecule. By "hybridize" is meant pair to form a double-stranded molecule between complementary polynucleotide sequences (e.g., a gene described herein), or portions thereof, under various conditions of stringency. (See, e.g., Wahl, G. M. and S. L. Berger (1987) Methods Enzymol. 152:399; Kimmel, A. R. (1987) Methods Enzymol. 152:507).

In **a third aspect,** the invention pertains to a computer readable medium comprising computer readable instructions stored thereon that when run on a computer perform a method according to the invention.

In **a fourth aspect,** the invention pertains a method for the diagnosis of a cancer disease in a subject, comprising the steps of detecting the absence or presence of a telomere fusion event in a sample of the subject using a method of the invention for the detection of a telomere fusion event according to the previous aspects.

In **a fifth aspect,** the invention pertains a method for the diagnosis of a cancer disease in a subject, comprising the steps of
- Providing a biological sample of the subject to be diagnosed;
- Nucleic acid sequencing the biological sample to obtain a dataset of nucleic acid sequencing reads;
- Performing a method according to the invention for the detection of a telomere fusion event with the dataset of nucleic acid sequencing reads of (b) in order to detect the presence or absence of at least one indicator sequencing read in the dataset of nucleic acid sequencing reads;

Wherein the presence of the at least one indicator sequencing read indicates the presence of a cancer disease characterized by the presence of a telomere fusion event in the subject.

The diagnostic method of the invention may be preferably performed on a biological sample which is selected from a tissue sample, such as a tumour sample, or a liquid sample, such as blood, serum, plasma, saliva, urine, smear or stool.

A cancer disease to be diagnosed in context of the invention is preferably a disease associated with the presence of telomere fusion, preferably of the alternative lengthening of telomeres (ALT) pathway. The method may thus comprise an additional step of determining any of the following: number of pure ALT-TFs, the total number of ALT-TFs, the length of the breakpoint sequence for each TF, and the abundance of the TVRs TGAGGG and TTAGGG.

Preferably in some embodiments a cancer to be diagnosed by the invention may be a cancer previously not associated with telomere fusion event, since there might be cancer diseases for which such association was not known. The presence of the telomere fusion events as detected in context of the invention are, however, in any case indicative for the presence or a high likelihood of the presence of a cancer disease.

The term "cancer", as used herein, refers to a disease characterized by uncontrolled cell division (or by an increase of survival or apoptosis resistance) and by the ability of such cells to invade other neighbouring tissues (invasion) and spread to other areas of the body where the cells are not normally located (metastasis) through the lymphatic and blood vessels, circulate through the bloodstream, and then invade normal tissues elsewhere in the body. Depending on whether or not they can spread by invasion and metastasis, tumours are classified as being either benign or malignant: benign tumours are tumours that cannot spread by invasion or metastasis, i.e., they only grow locally; whereas malignant tumours are tumours that are capable of spreading by invasion and metastasis. Biological processes known to be related to cancer include angiogenesis, immune cell infiltration, cell migration and metastasis. As used herein, the term cancer includes, but is not limited to, the following types of cancer: breast cancer; biliary tract cancer; bladder cancer; brain cancer including glioblastomas and medulloblastomas; cervical cancer; choriocarcinoma; colon cancer; endometrial cancer; esophageal cancer; gastric cancer; hematological neoplasms including acute lymphocytic and myelogenous leukemia; T-cell acute lymphoblastic leukemia/lymphoma; hairy cell leukemia; chronic myelogenous leukemia, multiple myeloma; AIDS-associated leukemias and adult T-cell leukemia/lymphoma; intraepithelial neoplasms including Bowen's disease and Paget's disease; liver cancer; lung cancer; lymphomas including Hodgkin's disease and lymphocytic lymphomas; neuroblastomas; oral cancer including squamous cell carcinoma; ovarian cancer including those arising from epithelial cells, stromal cells, germ cells and mesenchymal cells; pancreatic cancer; prostate cancer; rectal cancer; sarcomas including leiomyosarcoma, rhabdomyosarcoma, liposarcoma, fibrosarcoma, and osteosarcoma; skin cancer including melanoma, Merkel cell carcinoma, Kaposi's sarcoma, basal cell carcinoma, and squamous cell cancer; testicular cancer including germinal tumours such as seminoma, non-seminoma (teratomas, choriocarcinomas), stromal tumors, and germ cell tumors; thyroid cancer including thyroid adenocarcinoma and medullar carcinoma; and renal cancer including adenocarcinoma and Wilms tumor.

In another aspect, the in vitro method of the present invention is useful in monitoring effectiveness of therapeutics or in screening for drug candidates affecting the formation of telomere fusions. The ability to monitor telomere characteristics can provide a window for examining the effectiveness of particular therapies and pharmacological agents. The drug responsiveness of a disease state to a particular therapy in an individual may be determined by the in vitro method of the present disclosure, wherein shorter telomere length correlates with better drug efficacy. For example, the present disclosure also relates to the monitoring of the effectiveness of cancer therapy since the proliferative potential of cells is related to the maintenance of telomere integrity.

In accordance with the invention, the method may further comprise a subsequent step of characterizing the tumour, for example by detecting one or more specific tumour marker in the biological sample, and/or the dataset of nucleic acid sequencing reads.

One further additional aspect of the invention pertains to a method of monitoring progression of a disease, or monitoring the occurrence of a relapse of a cancer disease in a subject, the method comprising the steps of detecting the occurrence, and optionally quantification of the occurrence, of telomere fusion events in a sample of the subject, wherein the increased occurrence of telomere fusion events, such as an increased presence of indicator nucleic acids in the sample, compared to a sample obtained at an earlier time point, indicates a relapse in the subject.

The terms "of the [present] invention", "in accordance with the invention", "according to the invention" and the like, as used herein are intended to refer to all aspects and embodiments of the invention described and/or claimed herein.

As used herein, the term "comprising" is to be construed as encompassing both "including" and "consisting of', both meanings being specifically intended, and hence individually disclosed embodiments in accordance with the present invention. Where used herein, "and/or" is to be taken as specific disclosure of each of the two specified features or components with or without the other. For example, "A and/or B" is to be taken as specific disclosure of each of (i) A, (ii) B and (iii) A and B, just as if each is set out individually herein. In the context of the present invention, the terms "about" and "approximately" denote an interval of accuracy that the person skilled in the art will understand to still ensure the technical effect of the feature in question. The term typically indicates deviation from the indicated numerical value by ±20%, ±15%, ±10%, and for example ±5%. As will be appreciated by the person of ordinary skill, the specific such deviation for a numerical value for a given technical effect will depend on the nature of the technical effect. For example, a natural or biological technical effect may generally have a larger such deviation than one for a man-made or engineering technical effect. As will be appreciated by the person of ordinary skill, the specific such deviation for a numerical value for a given technical effect will depend on the nature of the technical effect. For example, a natural or biological technical effect may generally have a larger such deviation than one for a man-made or engineering technical effect. Where an indefinite or definite article is used when referring to a singular noun, e.g. "a", "an" or "the", this includes a plural of that noun unless something else is specifically stated.

It is to be understood that application of the teachings of the present invention to a specific problem or environment, and the inclusion of variations of the present invention or additional features thereto (such as further aspects and embodiments), will be within the capabilities of one having ordinary skill in the art in light of the teachings contained herein.

Unless context dictates otherwise, the descriptions and definitions of the features set out above are not limited to any particular aspect or embodiment of the invention and apply equally to all aspects and embodiments which are described.

All references, patents, and publications cited herein are hereby incorporated by reference in their entirety.

### BRIEF DESCRIPTION OF THE FIGURES AND SEQUENCES

The figures show:

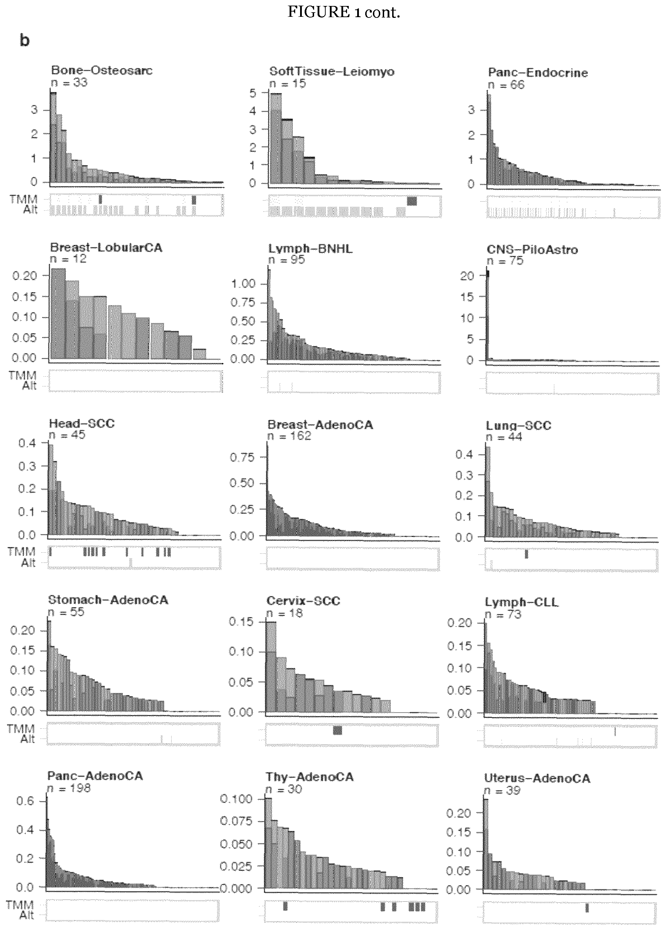

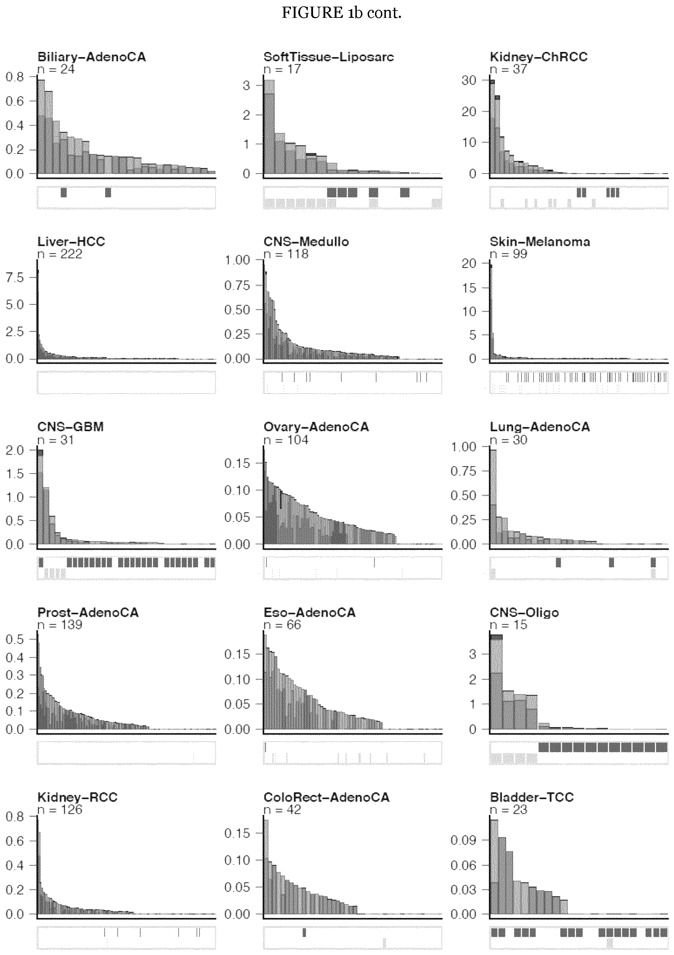

**Figure 1****: Landscape of telomere fusions in cancer. a**, Overview of the study design and schematic representation of the two types of telomere fusions (TFs) identified by TFDetector. **b**, TF rates across 30 cancer types from PCAWG. Outward fusions are shown in light grey, inward in dark grey, and circular (cases in which both inward and outward fusions are detected in reads from the same read pair) medium grey. Shades in the boxes below the bar plots represent the telomere maintenance mechanism (TMM) predictions reported by Sieverling et al. 2020 and de Nonneville et al. 2021. Only cancer types with at least 10 tumours are shown. The abbreviations used for the cancer types are as follows: Biliary-AdenoCA, biliary adenocarcinoma; Bladder-TCC, bladder transitional cell carcinoma; Bone-Benign, bone cartilaginous neoplasm, osteoblastoma and bone osteofibrous dysplasia; Bone-Epith, bone neoplasm, epithelioid; Bone-Osteosarc, sarcoma, bone; Breast-AdenoCA, breast adenocarcinoma; Breast-DCIS, breast ductal carcinoma in situ; Breast-LobularCA, breast lobular carcinoma; Cervix-AdenoCA, cervix adenocarcinoma; Cervix-SCC, cervix squamous cell carcinoma; CNS-GBM, central nervous system glioblastoma; CNS-Oligo, CNS oligodenroglioma; CNS-Medullo, CNS medulloblastoma; CNS-PiloAstro, CNS pilocytic astrocytoma; ColoRect-AdenoCA, colorectal adenocarcinoma; Eso-AdenoCA, esophagus adenocarcinoma; Head-SCC, head-and-neck squamous cell carcinoma; Kidney-ChRCC, kidney chromophobe renal cell carcinoma; Kidney-RCC, kidney renal cell carcinoma; Liver-HCC, liver hepatocellular carcinoma; Lung-AdenoCA, lung adenocarcinoma; Lung-SCC, lung squamous cell carcinoma; Lymph-CLL, lymphoid chronic lymphocytic leukemia; Lymph-BNHL, lymphoid mature B-cell lymphoma; Lymph-NOS, lymphoid not otherwise specified; Myeloid-AML, myeloid acute myeloid leukemia; Myeloid-MDS, myeloid myelodysplastic syndrome; Myeloid-MPN, myeloid myeloproliferative neoplasm; Ovary-AdenoCA, ovary adenocarcinoma; Panc-AdenoCA, pancreatic adenocarcinoma; Panc-Endocrine, pancreatic neuroendocrine tumor; Prost-AdenoCA, prostate adenocarcinoma; Skin-Melanoma, skin melanoma; SoftTissue-Leiomyo, leiomyosarcoma, soft tissue; SoftTissue-Liposarc, liposarcoma, soft tissue; Stomach-AdenoCA, stomach adenocarcinoma; Thy-AdenoCA, thyroid low-grade adenocarcinoma; and Uterus-AdenoCA, uterus adenocarcinoma.

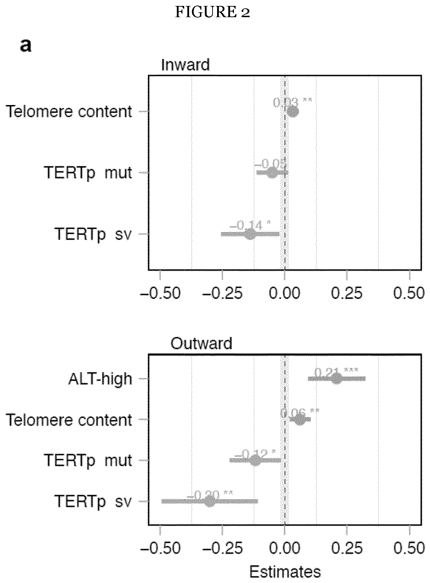

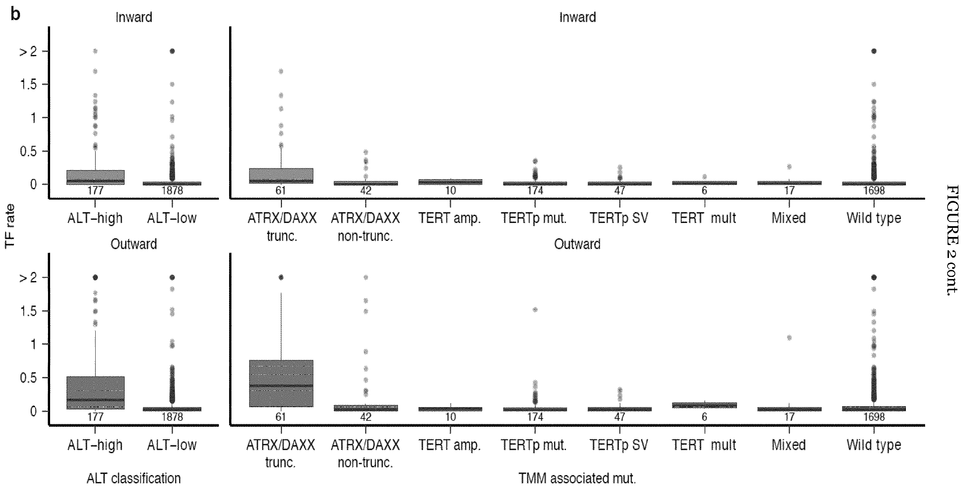

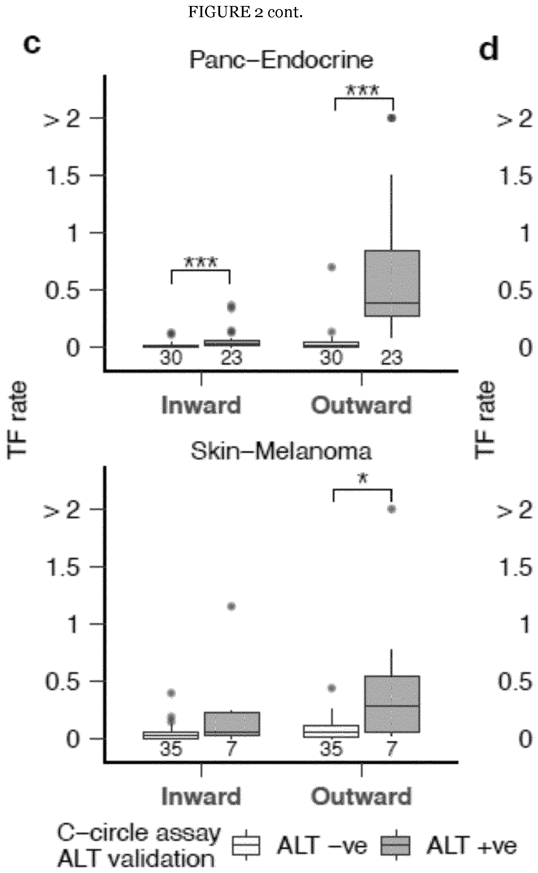

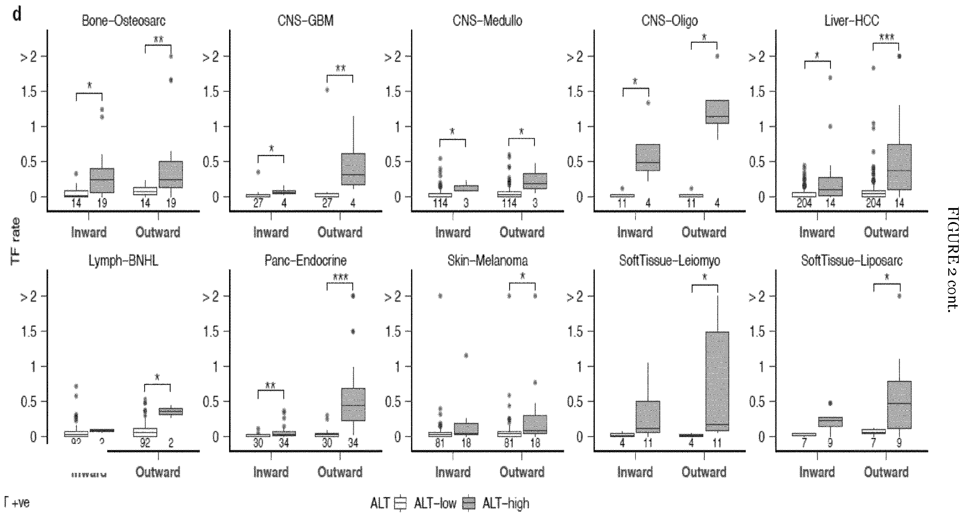

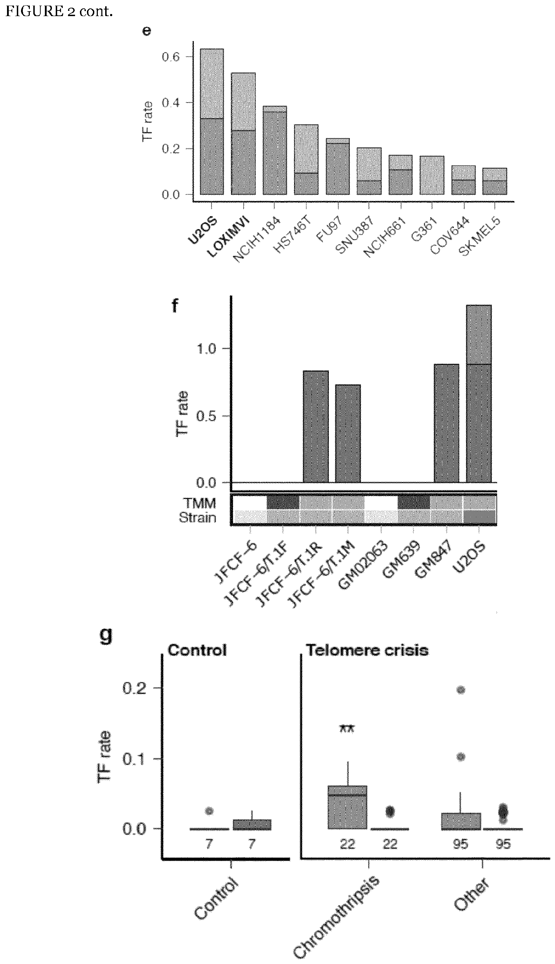

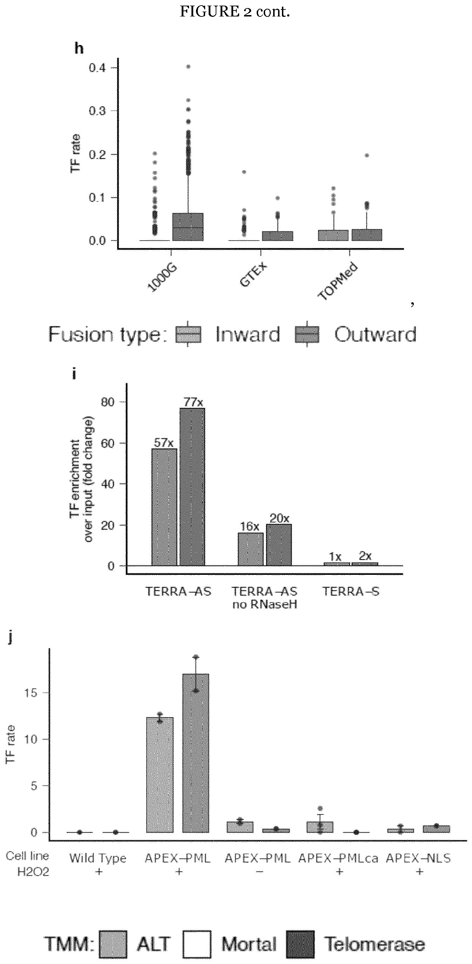

**Figure 2****: TFs are generated by the activity of the ALT pathway. a,** Coefficient values estimated using linear regression analysis and variable selection for the covariates with the strongest positive and negative association with TF rates. For this analysis we used the ALT status classification reported by de Nonneville et al 2021. **b**, Rates of inward and outward fusions in PCAWG tumours grouped by ALT status predictions (de Nonneville et al. 2021), and TMM-associated mutations (Sieverling et al. 2020). **c**, Comparison of TF rates between tumours positive and negative for the C-circle assay. **d**, TF rates estimated for PCAWG tumours grouped by ALT status predictions across selected cancer types. **e**, Top 10 cancer cell lines from the CCLE with the highest TF rates. ALT cell lines are indicated in bold type. **f**, TF rates in mortal cell strains before and after transformation by mechanisms requiring telomerase or ALT. **g**, TF rates detected in RPE-1 cell lines before (control) and after induction of telomere crisis with doxycycline. **h**, TF rates detected in 1000G, GTEx and TOPMed samples. **i**, Fold changes in TF rates over the control estimated using CHIRT-seq data from mouse embryonic stem cells treated with TERRA-AS, TERRA-AS without RNase H, and TERRA-S. j, TF rates estimated using ALaP data generated using different conditions of APEX knock-in and peroxidase (H2O2). In all panels ^{∗∗∗}*P* < 0.001; ^{∗∗}*P* < 0.01; ^{∗}*P* < 0.05, Wilcoxon rank-sum tests after FDR correction. Box plots show the median, first and third quartiles (boxes), and the whiskers encompass observations within a distance of 1.5× the interquartile range from the first and third quartiles.

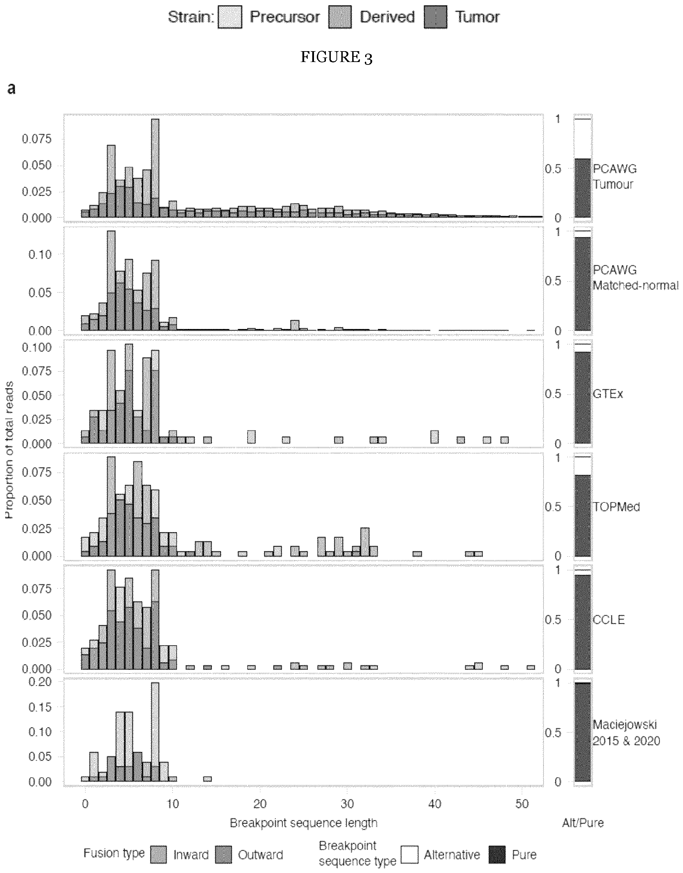

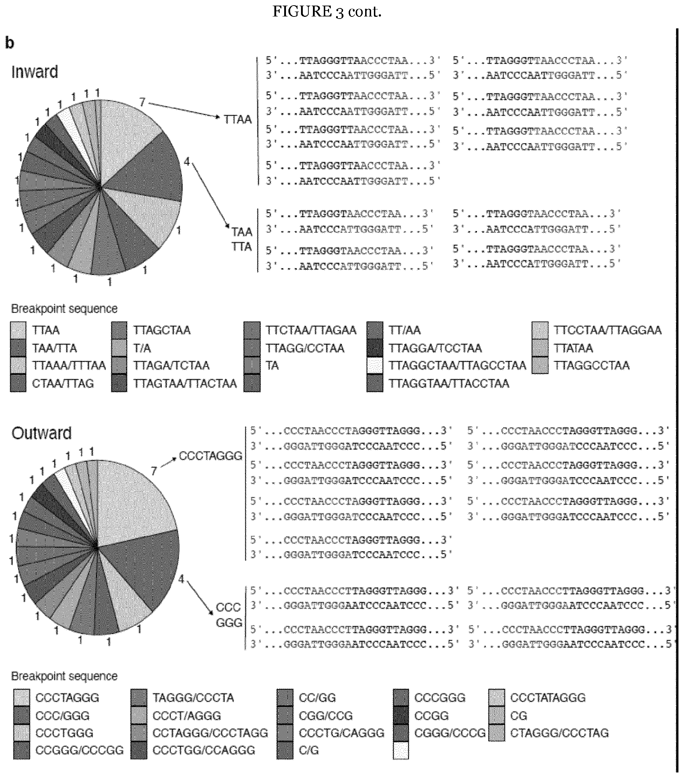

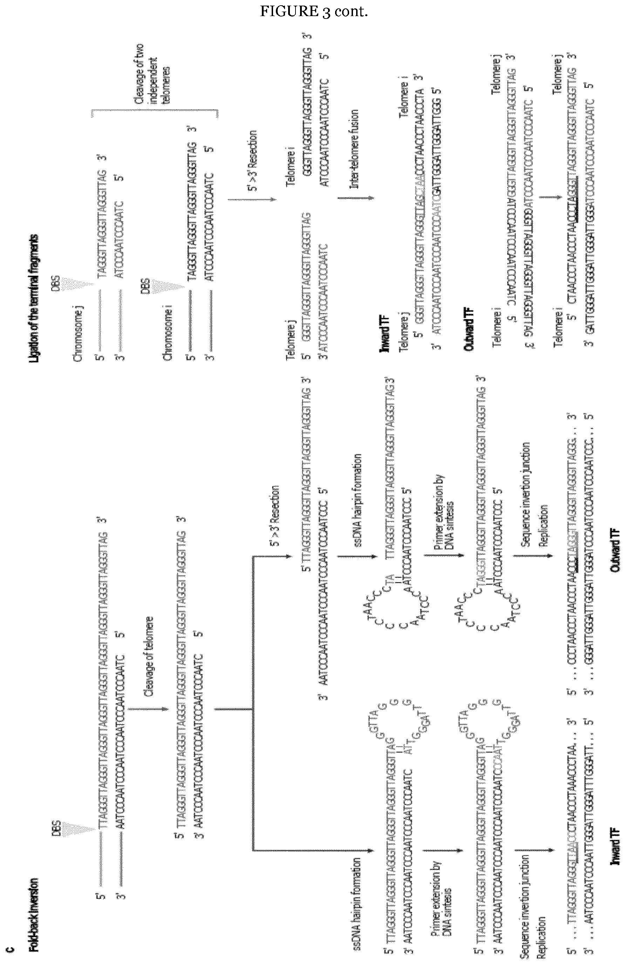

**Figure 3****: Mechanism of ALT-TF formation. a,** Breakpoint sequence length distribution. Inward and outward fusions are shown in red and blue, respectively. The bars on the right show the fraction of TFs classified as pure (black) or alternative (white). **b,** Pie chart showing the proportion of the distinct breakpoint sequences observed in pure TFs detected in PCAWG tumours. The numbers around the pie charts represent the number of combinations of circular permutations of repeat motifs that can generate originate each breakpoint sequence. The legend reports the breakpoint sequences in both strands unless they are identical (e.g., TTAA). The most represented breakpoint sequences are indicated. **c**, Proposed mechanisms for ATL-TF formation. ALT-TFs are generated through an intra-telomeric fold-back inversion after a double-strand break (left), or by the ligation of terminal telomere fragments after double-strand breaks (right).

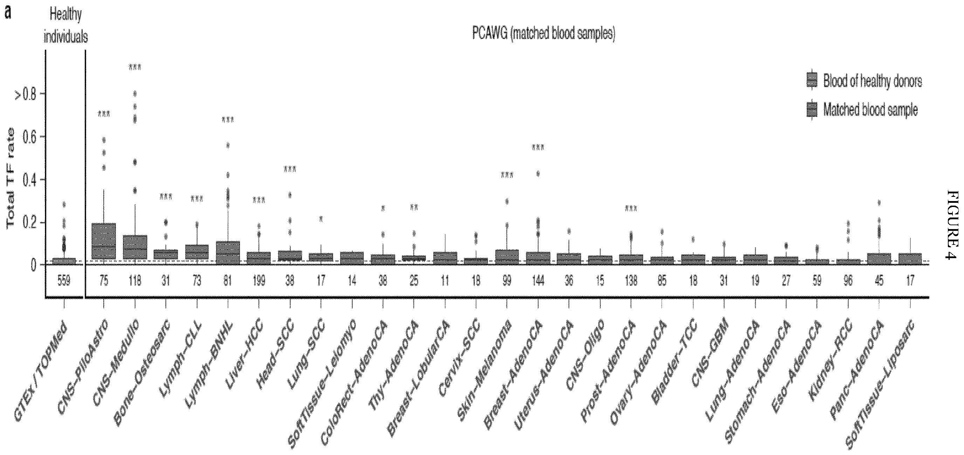

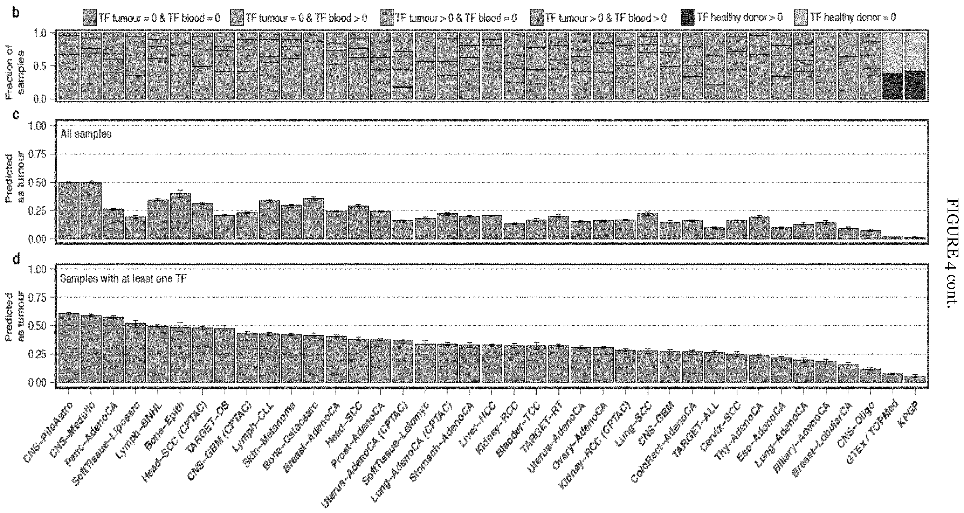

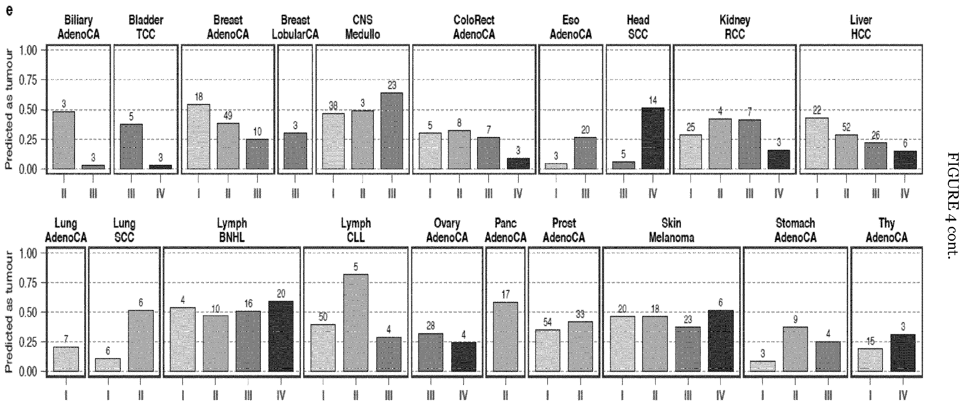

**Figure 4****: ALT-TFs detected in blood samples enable cancer detection. a,** TF rates in blood samples from healthy individuals from GTEx and TOPMed (green) and matched blood samples from PCAWG cohort (orange). **b**, Proportion of samples with at least 1 TF in the tumour and matched blood sample (shown in purple), at least 1 TF in blood but no TFs in the tumour sample (green), at least 1 TF in the tumour sample but no TFs in blood (blue), and no TFs detected in either the tumour or blood sample (red). **c**, Proportion of samples (mean value +/-95% confidence interval computed across 100 bootstrap resamples) predicted as cancer across diverse cancer types. Samples with no TFs in blood are included in this plot. **d**, Same as (**d**) but showing the results for samples with at least 1 TF in blood only. **e**, Fraction of PCAWG cases correctly classified as cancer stratified according to cancer stage. Predictions were computed using 100 Random Forest models trained on features of the TFs detected in WGS data for matched blood samples from PCAWG as well as blood samples from GTEx and TOPMed, which were used as controls. Only samples with at least 1 TF in blood were used for training. The number on top of each bar indicates the number of tumours of each type and stage. For each cancer type, only stages with at least 3 samples were included.

The sequences show:
SEQ ID NO: 1 shows GTTAGGGTTAGGGTTA
SEQ ID NO: 2 shows CCCTAACCCTAACCCTAA
SEQ ID NO: 3 shows CCCTAACCC**TA**GGGTTAGGG
SEQ ID NO: 4 shows CCCTAACCCTTAGGGTTAGGG
SEQ ID NO: 5 shows TTAGGGTTAACCCTAA
SEQ ID NO: 6 shows TTAGGGTAACCCTAA

### EXAMPLES

Certain aspects and embodiments of the invention will now be illustrated by way of example and with reference to the description, figures and tables set out herein. Such examples of the methods, uses and other aspects of the present invention are representative only, and should not be taken to limit the scope of the present invention to only such representative examples.

The examples show:

### Example 1: Pan-cancer landscape of telomere fusions

To detect TFs in sequencing data, the inventors developed TFDetector (Fig. 1a). In brief, TFDetector identifies sequencing reads containing at least two consecutive TTAGGG and two consecutive CCCTAA telomere sequences, allowing for mismatches to account for the variation observed in telomeric repeats in humans^{19,20} (Fig. 1). First the human reference genome was scanned to identify regions containing telomere fusion-like patterns, which could be misinterpreted as somatic (Methods). The analysis revealed the relic of an ancestral fusion in chromosome 2²¹, and a region in chromosome 9 containing 2 sets of telomeric repeats flanked by high complexity sequences, which the inventors term "chromosome 9 endogenous fusion".

To characterize the patterns and rates of somatic TFs across diverse cancer types, the inventors applied TFDetector to 2071 matched tumour and normal sample pairs from the Pan-Cancer Analysis of Whole Genomes (PCAWG) project that passed the QC criteria (Methods). To enable comparison of the relative number of TFs across samples, the inventors computed a telomere fusion rate for each tumour after correcting for tumour purity, sequencing depth, and read length. The inventors identified two distinct TF patterns, which differ in the relative position of the sets of TTAGGG and CCCTAA repeats (Fig. 1a). A first pattern, which is termed "inward TF", is characterized by 5'-TTAGGG-3' repeats followed by 5'-CCCTAA-3' repeats, which is the expected genomic footprint of end-to-end TFs^{1,13}. Unexpectedly, the inventors also found a second pattern characterized by 5'-CCCTAA-3' repeats followed by 5'-TTAGGG-3' repeats, which is termed "outward TF" (Fig. 1a), and represent a novel class of structural variation. In addition, the inventors found read pairs where a read in the pair contained an inward TF and the other an outward TF, which were classified as circular (in-out) TFs.

Both outward and inward TFs were detected across diverse cancer types, but rates varied markedly within and across tumour types (Fig. 1b). The highest TF rates were observed in osteosarcomas (Bone-Osteosarc), leiomyosarcomas (SoftTissue-Leiomyo), and pancreatic neuroendocrine tumours (Panc-Endocrine). The lowest frequencies were observed in thyroid adenocarcinomas (Thy-AdenoCA), renal cell-carcinomas (Kidney-RCC), and uterine adenocarcinomas (Uterus-AdenoCA). These results indicate that somatic TFs, including the novel type of outward fusions the inventors report here, are pervasive across diverse cancer types.

### Example 2: The ALT pathway is mechanistically linked with the formation of telomere fusions

Next, the inventors sought to determine the molecular mechanisms implicated in the generation of TFs. To this aim, the inventors regressed the observed rates of TFs on the mutation status of *ATRX, DAXX* and *TP₅₃*, telomere content, point mutations and structural variants in the TERTpromoter, expression values of *TERT* and TERRA, and a binary category indicating the ALT status of each tumour predicted using two previously published classifiers^{19,22} (Methods). Our analysis revealed a strong association between the activation of the ALT pathway and the rate of TFs, with the strongest effect size observed for outward TFs (P < 0.05; Fig. 2a,b and Extended Data Fig. 2a). However, alterations of the *TERT* promoter were negatively correlated with both inward and outward fusion rates, with the highest effect size for outward TFs (Fig. 2a). The association of TF rates with telomere content, TERRA expression, and *TP₅₃* mutations was also significant, although of a modest effect size (P < 0.001, ANOVA, Supplementary Table 3).

To investigate the association between the ALT pathway and TF formation, the inventors first compared the rate of TFs between tumours positive and negative for C-circles, an ALT marker^{19,22}. For this analysis, the inventors focused on published data for 42 skin melanomas and 53 pancreatic neuroendocrine tumours, which are also part of the PCAWG cohort. ALT tumours showed significantly higher rates of TFs in the pancreatic neuroendocrine tumour set (P < 0.001, two-tailed Mann-Whitney test; Fig. 2c). A similar trend was observed for skin melanomas, although only outward fusion rates reached significance (Fig. 2c). Next, the inventors extended this analysis to the entire cohort by comparing the TF rates between tumours with high and low ALT-probability scores (ALT-low vs ALT-high)²² on a per cancer type basis (Fig. 2d). Overall, TF rates, in particular for outward fusions, were significantly higher in cancer types classified as ALT-high (FDR-corrected P < 0.1, two-tailed Mann-Whitney test; Fig. 2d, see also Fig 1b).

To test whether TF fusions are enriched in ALT cancers, the inventors analysed whole-genome sequencing data for 306 cancer cell lines from the Cancer Cell Line Encyclopedia²³. Consistent with the observations in primary tumours, cell lines used as models of ALT, such as the osteosarcoma cell line U2OS and the melanoma cell line LOXIMVI, showed the highest rates of both inward and outward TFs (Fig. 2e, Extended Data Fig. 1i). Analysis of PacBio long-read sequencing data for the ALT breast cancer cell line SK-BR-3²⁴ also revealed an enrichment of outward fusions in this line as compared to the non-ALT cell lines COLO289T, HCT116, KM12, SW620 and SW837, for which long-read sequencing data were also available^{25,26} (Extended Data Fig. 2b-c; Supplementary Fig. 2 for examples).

To assess whether TFs are specifically associated with the ALT pathway, the inventors analyzed the genomes of mortal cell strains before and after transformation by mechanisms requiring telomerase or ALT²⁷. The genomes of parental mortal strains JFCF-6 and GM02063, as well as telomerase-positive strains JFCF-6/T.1F and GM639, did not contain outward TFs (Fig. 2f). In contrast, ALT-derived strains JFCF-6/T.1R, JFCF-6/T.1M and GM847 show a comparable outward TF fusion rate to the prototypical ALT cell line U2OS (Fig. 2f). Therefore, the presence of outward TFs in ALT derived-strains but not in telomerase-positive strains indicates that ALT activation leads to the formation of outward TFs. In addition, the inventors analysed TF rates in whole-genome sequencing data from hTERT-expressing retinal pigment epithelial (RPE-1) cells sequenced after the induction of telomere crisis using a dox-inducible dominant negative allele of *TRF2*^{9,10}*.* Compared to the control samples sequenced before induction of telomere crisis, the inventors detected a high rate of inward TFs consistent with the presence of end-to-end fusions (P < 0.05, two-tailed Mann-Whitney test, Fig. 2g, Extended Data Fig. 1j), thus lending further support to the mechanistic association between ALT activity and the formation of outward TFs. Consistent with the activation of the ALT pathway in cells immortalized by the Epstein-Barr virus in *vitro*^{28,29}, the inventors also detected high rates of outward TFs in 2490 Epstein-Barr virus-immortalized B cell lines from the 1000G project (Fig. 2h).

To further test the association between TFs and ALT activity, the inventors used Random Forest classification to predict the ALT status of tumours using the rates and features of TFs as covariates, and the set of tumours with C-circle assay data as the training set (Methods). Variable importance analysis using the best performing classifier (AUC=0.93) identified variables encoding the rate and breakpoint sequences of TFs as the most predictive, followed by the proportion of the telomere variant repeats (TVR) GTAGGG and CCCTAG, which were previously shown to be enriched in ALT tumours³⁰.

Together, these results mechanistically link the activity of the ALT pathway with the generation of somatic TFs. Therefore, the inventors term inward and outward fusions ALT-associated TFs (ALT-TFs).

### Example 3: ALT-TFs bind to TERRA and localize to APBs

The inventors next sought to determine the association of ALT-TFs with molecules involved in telomere maintenance and their cellular localization. Our regression expression analysis of the PCAWG data set indicates that tumours enriched in TFs present elevated levels of TERRA, a long non-coding RNA transcribed from telomeres^{31,32}. Previous genomic and cytological studies demonstrated a preferential association of TERRA transcripts to telomeres³³. To assess whether TERRA also associates with TFs, the inventors searched for inward and outward TFs in reads containing TERRA-binding sites. Specifically, the inventors analyzed reads from CHIRT-seq, an immunoprecipitation protocol that specifically captures TERRA-binding sites using an anti-sense biotinylated TERRA transcript (TERRA-AS) as bait³⁴. Targets of the TERRA-AS bait are then treated with RNase H to elute DNA containing TERRA binding sites followed by sequencing. By analyzing CHIRT-seq data sets from mouse embryonic stem cells³⁴, the inventors observed a 57-fold and 77-fold enrichment of inward and outward TFs, respectively, over the input using the TERRA-AS oligo probe (Fig. 2i). However, a modest enrichment was observed when the TERRA-AS not treated with RNase H or the TERRA sense transcript (TERRA-S) were used (Fig. 2i). These results indicate that TERRA binds to inward and outward TFs.

TERRA transcripts can be found in a subtype of promyelocytic leukaemia nuclear bodies (PML-NB) termed ALT-associated PML-Bodies (APBs)³⁵. Because TFs bind to TERRA, the inventors hypothesized that inward and/or outward fusions might locate to APBs. Given that PML-NBs, including APBs, are insoluble³⁶, a standard ChIP-seq for PML cannot be used to analyze whether TFs are present in APBs. To overcome PML-NBs accessibility problems, Kurihara *et al.* recently developed an assay called ALaP³⁷, for APEX-mediated chromatin labeling and purification by knocking in APEX, an engineered peroxidase, into the Pml locus to tag PML-NB partners in an H₂O₂-dependent manner. Applying ALaP in mESCs, PML-NBs bodies were found to be highly enriched in ALT-related proteins, such as DAXX and ATRX, as well as in telomere sequences. Here, to test this hypothesis, the inventors searched for TFs in ALaP genomic pull-downs and found a strong enrichment of both inward and outward TFs (P < 0.05, two-tailed Mann-Whitney test; Fig. 2j). In addition, the inventors found that negative controls, i.e., APEX-PMLs not-treated with H₂O₂ or APEX variants that do not form PML-NBs, rarely contain TFs (Fig. 2j). Therefore, these results indicate that APBs are a preferential location for ALT-TFs.

### Example 4: Short DNA fragments contain ALT-TFs

Besides APBs, another feature of ALT+ cells is their elevated levels of extrachromosomal telomeric DNA (ECT-DNA). Interestingly, most ECT-DNAs in ALT+ cells localize to APBs³⁸. As ALT-TFs also localize to APBs, it is conceivable that ECT-DNAs exert as substrates for the formation of ALT-TF. If this was the case, the ALT-TF formation would result in short, fused ECT-DNA fragments rather than fused chromosomes. To test this hypothesis, the inventors inferred the fragment size for read pairs with ALT-TF or chr9 endogenous fusions in which both mates support the same breakpoint sequence. The inventors found a significant enrichment of ALT-TFs in DNA fragments shorter than the insert size in a set of cancer types with high ALT-TF rates, such as melanomas, osteosarcomas, and glioblastomas (FDR-corrected P < 0.1; Chi-square test; Supplementary Table 4). Together, these results indicate that ALT-TFs might originate from the fusion of small fragments.

### Example 5: Sequence specificity at the telomere fusion point

The inventors next analyzed the set of sequences at the fusion point in PCAWG tumours. TFs with breakpoint sequences in the set of all possible circular permutations of TTAGGG and CCCTAA sequences were classified as pure (59% of TFs), whereas fusions with complex breakpoint sequences longer than 12bp were classified as alternative (41%; Fig. 3a, Supplementary Table 5 and Methods). In pure ALT-TFs, the inventors detected the entire set of possible permutations of telomere repeat motifs at fusion breakpoints, but not at similar frequencies (*P* < 0.05; chi-square test; Fig. 3b). In the case of outward TFs, the breakpoint sequence ^{5**'**}...CCCTAACCC**TA**GGGTTAGGG...^{3'} was the most abundant (22% of pure TFs) followed by ^{5'}...CCCTAACCC**TTA**GGGTTAGGG...^{3'} (16%). Interestingly, these two breakpoint sequences can be generated by the ligation of 7 and 4 combinations of telomeric repeats, respectively while the other breakpoint sequences detected can only be generated by the combination of two specific telomeric repeat sequences (Fig. 3b and Extended Fig. 3). In addition, these two sequences are the only ones in the entire set of breakpoint sequences in outward TFs with microhomology at the fusion point. In the case of inward TFs, the ^{5'}...TTAGGG**TTAA**CCCTAA...^{3'} sequence was the most abundant (14% of pure TFs) followed by ^{5'}...TTAGGG**TAA**CCCTAA...^{3'} (14%). The inventors also detected the TTAGCTAA sequence in 7% of pure TFs, which could be generated by the end-to-end fusion of two telomeres. In fact, the inventors detected this sequence in inward TFs at high frequency in cell lines induced to undergo telomere crisis through inactivation of *TRF2*¹³ (Fig. 2g and Supplementary Fig. 4). Similar to outward fusions, TTAA and TAA are the only breakpoint sequences in inward fusions that can be created by the ligation of several combinations of telomeric repeats and contain microhomology at the fusion junction (Fig. 3b). As microhomology facilitates ligation, the inventors conclude that microhomology at the fusion point also contributes to explain differences in the frequency of specific breakpoint sequences in both outward and inward TFs.

### Example 6: ALT-TFs are generated through the repair of double-strand breaks by an intra- or an inter-telomeric mechanism

Our previous analysis suggests that ALT-TFs are generated at APBs preferentially when telomeric fragments with microhomology in their ends fuse. Therefore, the inventors postulate two non-exclusive mechanisms of ALT-TF formation (Fig. 3c). First, a double-strand break in a telomere can be repaired through an intra-telomeric fold-back inversion. Specifically, end resection of a double-strand break would facilitate the formation of a hairpin loop when the 3' end of a telomere strand folds back to anneal its complementary strand through microhomology. Then, DNA synthesis would fill the gap to complete the capping of the hairpin. Finally, replication of the hairpin would create an inward or an outward fusion depending on the 3' end telomeric strand that folds back: ...(TTAGGG)ₙ...^{3'} fold-back would create an inward fusion and ...(CCCTAA)ₙ...^{3'} fold-back would create and outward fusion. Secondly, ALT-TFs can also be generated through the ligation of the terminal fragments upon double-strand DNA breaks in telomeres (Fig. 3c). Specifically, an inter-telomeric mechanism would occur when two telomeres covalently fuse in ^{5'} ...(TTAGGG)ₙ...-...(CCCTAA)ₙ...^{3'} orientation to create an inward fusion, or in ^{5'} ...(CCCTAA)ₙ ...-...(TTAGGG)ₙ...^{3'} orientation to create an outward fusion. Outward fusions are only feasible when telomeric fragments join from the broken ends produced after telomere trimming (Fig. 3c).

### Example 7: ALT-TFs are detected in blood and enable cancer detection

Given the high rate of ALT-TFs observed in tumours of diverse origin, the inventors hypothesized that ALT-TFs could also be detected in blood samples and used as biomarkers for liquid biopsy analysis. To test this hypothesis, the inventors applied TFDetector to blood samples from PCAWG (1604), the Genotype-Tissue Expression (GTEx; 255) project and Trans-Omics for Precision Medicine program (TOPMed; 304), respectively (Methods). Overall, blood samples from cancer patients showed a significantly higher rate of ALT-TFs, in particular of the outward type (FDR-corrected *P* < 0.1, two-tailed Mann-Whitney test; Fig. 4a and Extended Data Fig. 4).

Next, the inventors utilized Random Forest (RF) classification to model the probability that an individual has cancer based on the patterns of ALT-TFs detected in blood. For this analysis the inventors also included 438 blood samples from cancer patients from the Clinical Proteomic Tumour Analysis Consortium (CPTAC) cohort, 119 blood childhood cancers samples from The Therapeutically Applicable Research to Generate Effective Treatments (TARGET) program, and 99 blood samples from healthy individuals from Korean Personal Genome Project (KPGP)³⁹. In brief, each blood sample, from either a healthy donor or a cancer patient, was encoded by a vector recording 117 features of the ALT-TFs detected (Methods and Supplementary Table 6). By focusing on those blood samples with at least 1 ALT-TF (66.9% of cancer patients and 45.6% of controls, Fig. 4b), the inventors obtained high sensitivity for pilocytic astrocytomas (sensitivity: 0.61), medulloblastomas (0.59), pancreatic adenocarcinomas (0.58) and liposarcoma (0.52), and (Fig. 4c-d). The false positive rate was low (<8% of samples with TF > 0, which represents < 2% of all control samples, Supplementary Table 7) and the performance of the present classifier was comparable across cancer stages (Fig. 4e). The most predictive features included the number of pure ALT-TFs, the total number of ALT-TFs, the length of the breakpoint sequence, and the abundance of the TVRs TGAGGG and TTAGGG, which have been previously linked with ALT activity (Supplementary Fig. 5)^{19,22}. Notably, the inventors obtained a comparable sensitivity of detection even for non-ALT tumours (Supplementary Fig. 5d). This is consistent with studies reporting the coexistence of telomerase expression and ALT in the same cell populations in *vitro*^{40,41} and in primary tumours⁴²⁻⁴⁴. Together, these results indicate that the detection of somatic ALT-TFs in blood represents a highly specific biomarker for liquid biopsy analysis.

### REFERENCES

The references are:
1. Maciejowski, J. & Lange, T. de. Telomeres in cancer: tumour suppression and genome instability. Nat. Rev. Mol. Cell Biol. 2017 183 18, 175-186 (2017).
2. Barthel, F. P. et al. Systematic analysis of telomere length and somatic alterations in 31 cancer types. Nat. Genet. 49, 349-357 (2017).
3. Huang, F. W. et al. Highly recurrent TERT promoter mutations in human melanoma. Science (80-.).339, 957-959 (2013).
4. Horn, S. et al. TERT promoter mutations in familial and sporadic melanoma. Science (80-.).339, 959-961 (2013).
5. Peifer, M. et al. Telomerase activation by genomic rearrangements in high-risk neuroblastoma. Nature 526, 700-704 (2015).
6. Bryan, T. M., Englezou, A., Dalla-Pozza, L., Dunham, M. A. & Reddel, R. R. Evidence for an alternative mechanism for maintaining telomere length in human tumours and tumor-derived cell lines. Nat. Med. 1997 311 3, 1271-1274 (1997).
7. Blackburn, E. H., Greider, C. W. & Szostak, J. W. Telomeres and telomerase: the path from maize, Tetrahymena and yeast to human cancer and aging. Nat. Med. 12, 1133-8 (2006).
8. Umbreit, N. T. et al. Mechanisms generating cancer genome complexity from a single cell division error. Science (80-.).368, (2020).
9. Maciejowski, J., Li, Y., Bosco, N., Campbell, P. J. & de Lange, T. Chromothripsis and Kataegis Induced by Telomere Crisis. Cell 163, 1641-1654 (2015).
10. Maciejowski, J. et al. APOBEC3-dependent kataegis and TREX1-driven chromothripsis during telomere crisis. Nat. Genet. 52, 884-890 (2020).
11. Dewhurst, S. M. et al. Structural variant evolution after telomere crisis. Nat. Commun. 2021 121 12, 1-17 (2021).
12. Shoshani, O. et al. Chromothripsis drives the evolution of gene amplification in cancer. Nat. 2020 5917848 591, 137-141 (2020).
13. van Steensel, B., Smogorzewska, A. & de Lange, T. TRF2 protects human telomeres from end-to-end fusions. Cell 92, 401-13 (1998).
14. Stohr, B. A., Xu, L. & Blackburn, E. H. The terminal telomeric DNA sequence determines the mechanism of dysfunctional telomere fusion. Mol. Cell 39, 307-14 (2010).
15. Tusell, L., Pampalona, J., Soler, D., Frias, C. & Genesca, A. Different outcomes of telomere-dependent anaphase bridges. Biochem. Soc. Trans. 38, 1698-703 (2010).
16. Capper, R. et al. The nature of telomere fusion and a definition of the critical telomere length in human cells. Genes Dev. 21, 2495-508 (2007).
17. Tanaka, H. et al. Telomere fusions in early human breast carcinoma. Proc. Natl. Acad. Sci. U. S. A. 109, 14098-103 (2012).
18. Debode, F., Marien, A., Janssen, E., Bragard, C. & Berben, G. The influence of amplicon length on real-time PCR results. Biotechnologie 21, 3-11 (2017).
19. Sieverling, L. et al. Genomic footprints of activated telomere maintenance mechanisms in cancer. Nat. Commun. 11, 733 (2020).
20. Grigorev, K. et al. Haplotype Diversity and Sequence Heterogeneity of Human Telomeres. 1269-1279 (2020). doi:10.1101/2020-01-31.929307
21. IJdo, J. W., Baldini, A., Ward, D. C., Reeders, S. T. & Wells, R. A. Origin of human chromosome 2: an ancestral telomere-telomere fusion. Proc. Natl. Acad. Sci. U. S. A. 88, 9051 (1991).
22. de Nonneville, A. & Reddel, R. R. Alternative lengthening of telomeres is not synonymous with mutations in ATRX/DAXX. Nat. Commun. 12, 10-13 (2021).
23. Ghandi, M. et al. Next-generation characterization of the Cancer Cell Line Encyclopedia. Nature 1 (2019). doi:10.1038/s41586-019-1186-3
24. Aganezov, S. et al. Comprehensive analysis of structural variants in breast cancer genomes using single-molecule sequencing. Genome Res. 30, 1258-1273 (2020).
25. Valle-Inclan, J. E. et al. A multi-platform reference for somatic structural variation detection. bioRxiv 2020.10.15.340497 (2020). doi:10.1101/2020.10.15.340497
26. Wietmarschen, N. van et al. Repeat expansions confer WRN dependence in microsatellite-unstable cancers. Nat. 2020 5867828 586, 292-298 (2020).
27. Lee, M. et al. Telomere extension by telomerase and ALT generates variant repeats by mechanistically distinct processes. Nucleic Acids Res. 42, 1733-1746 (2014).
28. Kamranvar, S. A. & Masucci, M. G. Regulation of telomere homeostasis during epstein-barr virus infection and immortalization. Viruses 9, 1-15 (2017).
29. Reddel, R. R., Bryan, T. M., Colgin, L. M., Perrem, K. T. & Yeager, T. R. Alternative lengthening of telomeres in human cells. Radiat. Res. 155, 194-200 (2001).
30. Lee, M. et al. Telomere sequence content can be used to determine ALT activity in tumours. Nucleic Acids Res. 46, 4903-4918 (2018).
31. Luke, B. & Lingner, J. TERRA: telomeric repeat-containing RNA. EMBO J. 28, 2503-10 (2009).
32. Schoeftner, S. & Blasco, M. A. Chromatin regulation and non-coding RNAs at mammalian telomeres. Semin. Cell Dev. Biol. 21, 186-93 (2010).
33. Fernandes, R. V., Feretzaki, M. & Lingner, J. The makings of TERRA R-loops at chromosome ends. Cell Cycle 1-15 (2021). doi:10.1080/15384101.2021.1962638
34. Chu, H. P. et al. TERRA RNA Antagonizes ATRX and Protects Telomeres. Cell 170, 86-101.e16 (2017).
35. Arora, R. et al. RNaseH1 regulates TERRA-telomeric DNA hybrids and telomere maintenance in ALT tumour cells. Nat. Commun. 5, 5220 (2014).
36. Chang, K. S., Fan, Y. H., Andreeff, M., Liu, J. & Mu, Z. M. The PML gene encodes a phosphoprotein associated with the nuclear matrix. Blood 85, 3646-53 (1995).
37. Kurihara, M. et al. Genomic Profiling by ALaP-Seq Reveals Transcriptional Regulation by PML Bodies through DNMT3A Exclusion. Mol. Cell 78, 493-505.e8 (2020).
38. Loe, T. K. et al. Telomere length heterogeneity in ALT cells is maintained by PML-dependent localization of the BTR complex to telomeres. Genes Dev. 34, 650-662 (2020).
39. Kim, J. et al. KoVariome: Korean National Standard Reference Variome database of whole genomes with comprehensive SNV, indel, CNV, and SV analyses. Sci. Reports 2018 81 8, 1-14 (2018).
40. Perrem, K., Colgin, L. M., Neumann, A. A., Yeager, T. R. & Reddel, R. R. Coexistence of alternative lengthening of telomeres and telomerase in hTERT-transfected GM847 cells. Mol. Cell. Biol. 21, 3862-75 (2001).
41. Cerone, M. A., Londono-Vallejo, J. A. & Bacchetti, S. Telomere maintenance by telomerase and by recombination can coexist in human cells. Hum. Mol. Genet. 10, 1945-52 (2001).
42. Ulaner, G. A. et al. Absence of a telomere maintenance mechanism as a favorable prognostic factor in patients with osteosarcoma. Cancer Res. 63, 1759-63 (2003).
43. Hakin-Smith, V. et al. Alternative lengthening of telomeres and survival in patients with glioblastoma multiforme. Lancet 361, 836-838 (2003).
44. Xu, B., Peng, M. & Song, Q. The co-expression of telomerase and ALT pathway in human breast cancer tissues. Tumour Biol. 2013 355 35, 4087-4093 (2013).
45. Viswanath, P. et al. Non-invasive assessment of telomere maintenance mechanisms in brain tumors. Nat. Commun. 2021 121 12, 1-18 (2021).
46. Mukherjee, J. et al. A subset of PARP inhibitors induces lethal telomere fusion in ALT-dependent tumour cells. Sci. Transl. Med. 13, 7211 (2021).
47. Chen, B. et al. Dynamic Imaging of Genomic Loci in Living Human Cells by an Optimized CRISPR/Cas System. Cell 155, 1479-1491 (2013).
48. Liu, M. C. et al. Sensitive and specific multi-cancer detection and localization using methylation signatures in cell-free DNA. Ann. Oncol. 31, 745-759 (2020).
49. Zviran, A. et al. Genome-wide cell-free DNA mutational integration enables ultra-sensitive cancer monitoring. Nat. Med. 26, 1114-1124 (2020).
50. Killcoyne, S. et al. Genomic copy number predicts esophageal cancer years before transformation. Nat. Med. 26, 1726-1732 (2020).
51. Campbell, P. J. et al. Pan-cancer analysis of whole genomes. Nature 578, (2020).
52. Edwards, N. J. et al. The CPTAC Data Portal: A Resource for Cancer Proteomics Research. J. Proteome Res. 14, 2707-2713 (2015).
53. Rodriguez, H., Zenklusen, J. C., Staudt, L. M., Doroshow, J. H. & Lowy, D. R. The next horizon in precision oncology: Proteogenomics to inform cancer diagnosis and treatment. Cell 184, 1661-1670 (2021).
54. GTEx Consortium et al. Genetic effects on gene expression across human tissues. Nature 550, 204-213 (2017).
55. Auton, A. et al. A global reference for human genetic variation. Nature 526, 68-74 (2015).
56. Stoler, N. & Nekrutenko, A. Sequencing error profiles of Illumina sequencing instruments. NAR Genomics Bioinforma. 3, (2021).
57. Cortés-Ciriano, I. et al. Comprehensive analysis of chromothripsis in 2,658 human cancers using whole-genome sequencing. Nat. Genet. 52, 331-341 (2020).
58. Zapatka, M. et al. The landscape of viral associations in human cancers. Nat. Genet. 52, 320-330 (2020).

## Claims

1. **A method for the detection of the presence of at least one telomere fusion event**, the method comprising the steps of:
• Providing a biological sample containing nucleic acids which are chromosomal nucleic acids or nucleic acids derived from one or more chromosomes, such as extra chromosomal nucleic acids;
• Detecting in the biological sample the presence or absence of at least one indicator nucleic acid which is **characterized by** having a nucleic acid sequence comprising a first sequence stretch and a second sequence stretch on the same nucleic acid strand, wherein
• the first sequence-stretch is a sequence of at least 12 directly adjacent (or closely adjacent) nucleic acid base pairs (bp) within the sequence: GGGTTAGGGTTAGGGTTA (SEQ ID NO: 1), wherein the first sequence stretch may not comprise more than two, preferably no more than one, bp variation within this sequence;
• the second sequence-stretch is a sequence of at least 12 directly adjacent (or closely adjacent) nucleic acid bp within the sequence: CCCTAACCCTAACCCTAA (SEQ ID NO: 2), wherein the second sequence stretch may not comprise more than two, preferably no more than one, bp variation within this sequence;
wherein the presence of the at least one indicator nucleic acid sequence indicates the presence of the at least one telomere fusion event.

2. **A method for the detection of the presence of at least one telomere fusion event**, the method comprising the steps of:
• Providing a dataset of nucleic acid sequencing reads, wherein the dataset of nucleic acid sequencing reads is obtained by Sanger sequencing, next generation sequencing (NGS) or long-read sequencing of nucleic acids of nucleic acids derived from a cellular sample;
• Detecting within the dataset of nucleic acid sequencing reads the presence or absence of at least one indicator sequencing read which is **characterized by** having a nucleic acid sequence comprising a first sequence stretch and a second sequence stretch on the same strand, wherein
• the first sequence-stretch is a sequence of at least 12 directly adjacent (or closely adjacent) nucleic acid base pairs (bp) within the sequence: GGGTTAGGGTTAGGGTTA (SEQ ID NO: 1), wherein the first sequence stretch may not comprise more than two, preferably no more than one, bp variation within this sequence;
• the second sequence-stretch is a sequence of at least 12 directly adjacent (or closely adjacent) nucleic acid bp within the sequence: CCCTAACCCTAACCCTAA (SEQ ID NO: 2), wherein the second sequence stretch may not comprise more than two, preferably no more than one, bp variation within this sequence;
wherein the presence of the at least one indicator nucleic acid sequencing read indicates the presence of the at least one telomere fusion event.

3. The method of claim 1 or 2, wherein indicator nucleic acid or indicator nucleic acid sequencing read is further **characterized in that** the first sequence-stretch and second sequence-stretch are directly adjacent to each other, or are separated by an inserted sequence having a length of 1 to 50 nucleic acids.

4. The method of any one of claims 1 to 3, wherein if the indicator nucleic acid or indicator nucleic acid sequencing read is further **characterized in that** the first sequence stretch is in 5' position of the second sequence stretch, the presence of the at least one indicator nucleic acid or indicator nucleic acid sequencing read indicates the presence of the at least one inward telomere fusion event (according to figure 1); or wherein if the indicator nucleic acid or indicator nucleic acid sequencing read is further **characterized in that** the first sequence stretch is in 3' position of the second sequence stretch, the presence of the at least one indicator nucleic acid or indicator nucleic acid sequencing read indicates the presence of the at least one outward telomere fusion event (according to figure 1).

5. The method of any one of claims 1 to 4, wherein the telomere fusion is an ALTernative Telomere Fusion (ALT-TF).

6. The method of any one of claims 1 to 8, which is an *in-silico* and / or *in-vitro* method.

7. **A computer readable medium** comprising computer readable instructions stored thereon that when run on a computer perform a method according to any one of claims 1 to 6.

8. **A method for the diagnosis of a cancer disease in a subject,** comprising the steps of detecting the presence or absence of an indicator nucleic acid or indicator nucleic acid sequencing read in accordance with a method of any one of claims 1 to 6, wherein the presence of the at least one indicator sequencing read indicates the presence of a cancer disease **characterized by** the presence of a telomere fusion event in the subject.

9. The method according to claim 8, wherein the biological sample is selected from a tissue sample, such as a tumor sample, or a liquid sample, such as blood, serum, plasma, saliva, urine, smear or stool.

10. The method of claim 8 or 9, wherein the cancer disease is a disease associated with the presence of telomere fusion of the alternative lengthening of telomeres (ALT) pathway.

11. The method of any one of claims 8 to 10, wherein the method comprises an additional step of determining any of the following: number of pure ALT-TFs, the total number of ALT-TFs, the length of the breakpoint sequence for each TF, and the abundance of the TVRs TGAGGG and TTAGGG.

12. The method of any one of claim 8 to 11, further comprising a subsequent step of characterizing the tumor, for example by detecting one or more specific tumor marker in the biological sample, and/or the dataset of nucleic acid sequencing reads.
